# EUROPEAN PATENT SPECIFICATION

(11) **EP 2 121 001 B1**
(45) Date of publication and mention of the grant of the patent: **17.12.2014**
(21) Application number: 08725396.9
(22) Date of filing: 11.02.2008
(51) Int. Cl.: A61K 38/18, A61K 31/00, A61K 31/05, A61K 31/335, A61K 31/35, A61K 31/365, A61K 31/395, A61K 31/4015, A61P 25/00, A61P 25/28, A61K 45/00

(54) **THERAPEUTIC EFFECTS OF BRYOSTATINS ON ISCHEMIA/STROKE-INDUCED MEMORY IMPAIRMENT AND BRAIN INJURY**
THERAPEUTISCHE WIRKUNGEN VON BRYOSTATINEN AUF DURCH ISCHÄMIE/SCHLAGANFALL HERBEIGEFÜHRTE GEDÄCHTNISSTÖRUNGEN UND HIRNVERLETZUNGEN
EFFETS THÉRAPEUTIQUES DE BRYOSTATINES SUR L'ALTÉRATION DE LA MÉMOIRE INDUITE PAR UNE ISCHÉMIE/UN ACCIDENT VASCULAIRE CÉRÉBRAL ET UNE LÉSION CÉRÉBRALE

(30) Priority: 09.02.2007 US 900339 P; 24.05.2007 US 924662 P
(43) Date of publication of application: 25.11.2009
(60) Divisional application: 14000741.0
(73) Proprietor: Blanchette Rockefeller Neurosciences Institute, Morgantown, WV 26505-3409 (US)
(72) Inventor: SUN, Miao-kun, Gaithersburg, MD 20879 (US); ALKON, Daniel, L., Bethesda, Maryland 20817 (US)
(74) Representative: Kador & Partner
(86) International application number: PCT/US2008/001756
(87) International publication number: WO 2008/100450

(56) References cited:
- WO-A-2004/004641
- WO-A-2004/047857
- WO-A-2006/031337
- WO-A-2008/013573

## Description

### FIELD OF THE INVENTION

The present invention relates to a pharmaceutical composition for use in a medicament effective in the treatment of at least one symptom of stroke comprising a protein kinase C (PKC) activator.

### BACKGROUND OF THE INVENTION

### A. Stroke

A stroke, also known as cerebrovascular accident (CVA), is an acute neurological injury in which the blood supply to a part of the brain is interrupted. Blood supply to the brain may be interrupted in several ways, including occlusion (ischemic, embolic or thrombotic stroke) or blood-vessel rupture (hemorrhagic stroke). A stroke involves the sudden loss of neuronal function due to disturbance in cerebral perfusion. This disturbance in perfusion is commonly arterial, but can be venous.

The part of the brain with disturbed perfusion no longer receives adequate oxygen. This initiates the ischemic cascade which causes brain cells to die or be seriously damaged, impairing local brain function. Stroke is a medical emergency and can cause permanent neurologic damage or even death if not promptly diagnosed and treated. It is the third leading cause of death and the leading cause of adult disability in the United States and industrialized European nations. On average, a stroke occurs every 45 seconds and someone dies every 3 minutes. Of every 5 deaths from stroke, 2 occur in men and 3 in women.

Despite the medical emergency and the multiple agents that have been shown to be effective in arresting the pathological processes of cerebral ischemia in preclinical studies, thromobolytic therapy using rTPA is currently the only option available for the treatment of ischemic stroke. The treatment is designed to achieve early arterial recanalization, which is time-dependent (within 3 hours after the event to be effective). The effectiveness of rTPA and other potential agents for arresting infarct development, depends on early administration or even before the ischemic event, if possible. The narrow therapeutic time window in treating ischemic stroke leads to about only 5% of candidate patients receiving effective intravenous thrombolytic therapy.

Significant brain injury occurs in ischemic stroke after the immediate ischemic event. The "delayed" brain injury and cell death in cerebral ischemia/stroke is a well-established phenomenon, representing a therapeutic opportunity. Neurons in the infarction core of focal, severe stroke are immediately dead and cannot be saved by pharmacologic intervention. The ischemic penumbra, consisting of the brain tissue around the core in focal ischemic stroke, and the sensitive neurons/network in global cerebral ischemia, however, are maintained by a diminished blood supply. The damage to this penumbral brain tissue occurs in a "delayed" manner, starting 4-6 hours as the second phase or days and weeks later as the the so-called third phase, after cerebral ischemia/stroke. After an about 15 minute cerebral ischemia, for example, the hippocampal CA 1 pyramidal cells start to degenerate within 2-3 days, and reach the maximal extent of cell death a week after the ischemic event. The sensitive neuronal structures in global cerebral ischemia and the ischemic penumbra are "at-risk" tissues. Their salvage through intervention or further damage in the subsequent days or weeks determine dramatic differences in long-term disability.

The present invention provides a new therapeutic strategy comprising the transient, periodic or chronic administration of a PKC activator as defined in claim 1 to a subject suffering from cerebral ischemia/stroke over a broader therapeutic window after the ischemic event as defined in claim 1.

### B. Protein Kinase C

PKC has been identified as one of the largest gene families of non-receptor serine-threonine protein kinases. Since the discovery of PKC in the early eighties by Nishizuka and coworkers (Kikkawa et al. (1982) J. Biol. Chem. 257: 13341), and its identification as a major receptor for phorbol esters (Ashendel et al. (1983) Cancer Res., 43: 4333), a multitude of physiological signaling mechanisms have been ascribed to this enzyme. The intense interest in PKC stems from its unique ability to be activated *in vitro* by calcium and diacylglycerol (and its phorbol ester mimetics), an effector whose formation is coupled to phospholipid turnover by the action of growth and differentiation factors.

The activation of PKC has been shown to improve learning and memory. (WO 2002/087423; WO 2003/075930; US provisional patent application no. 60/287,721; US provisional patent application no. 60/362,081 ; US 2003/0171385 A1; and US 2004/0235889 A1). Prior to the present disclosure, however, the PKC-mediated improvement of learning and memory has not been recognized as a mechanism for the treatment of post-stroke memory deficits and brain injury. Also, the PKC activators disclosed herein, specifically those compounds that improve learning and memory, were not recognized as possessing brain function-restoring activity after cerebral ischemia/stroke.

Stroke therapy has historically been limited to few treatment options available. The only drug therapy currently available, for instance, consists of antithrombotics (thrombolytic therapy; such as intravenous injections of tissue plasminogen activator), which have to be administered within 3 hours of the ischemic event. Although many types of potential neuroprotectants have been tested in clinical trials, none has been approved for clinical use, because of ineffectiveness especially when used post-stroke or associated toxicity. The compounds presented in this invention disclosure were effective when the treatment was started 24 hours after the ischemia in the animal model at doses that have already been demonstrated to be well tolerated in humans (the bryostatin-1 doses). Compounds that target the protein kinase C (PKC) such as bryostatin-1 , a direct PKC activator, and methylcatechol diacetic acid, a derivative of methylcatechol, an enhancer or means of activating or mobilizing nerve growth factor (NGF), brain-derived neurotrophic factor (BDNF) or other neurotrophic factors, which is perhaps one of the PKC targets, have been found to have therapeutic value against brain injury and memory impairment induced with cerebral ischemia in rats (an animal stroke model). The development of these substances as therapeutic in the treatment of stroke is provided by this invention.

WO 2006/031337 discloses compositions comprising a combination of PKC activators and PKC inhibitors and methods to modulate α-secretase activity to improve or enhance cognitive ability and/or reduce neuro-degeneration in individuals suffering from diseases that impair cognitive ability. Macrocyclic lactones are preferred PKC activators for use in said compositions.

WO 2004/004641 discloses a method for enhancing cognitive ability in a human or animal comprising administering to said human or animal a PKC activator in an amount effective for enhancing cognitive ability in a pharmaceutically acceptable carrier, wherein the PKC activator is a macrocyclic lactone, a benzolactam, a pyrollidone or a combination thereof. The PKC activator is effective to treat cognitive impairment of a neurological disease or disorder associated with brain damage caused by stroke, an anesthetic accident, head trauma, hypoglycemia, carbon monoxide poisoning, lithium intoxication or a vitamin deficiency.

WO 2004/047857 relates to the use of fibroblast growth factor 18 (FGF-18) to improve learning and memory. Further disclosed are methods of measuring expression levels of FGF-18 and the central nervous system and the use of such measurements for diagnostic purposes. The disclosed compounds and methods are expected to be useful in the areas of associative learning, consolidated memory, drug development and analysis and in the treatment of certain diseases associated with impaired hypocampal function such as dementia due to Alzheimer's disease.

WO 2008/013573 relates to methods of stimulating cellular growth, synaptic remodeling and consolidation of long-term memory comprising the step of contacting an effective amount of a PKC activator with a protein kinase C (PKC) in a subject identified with memory loss, slowing or reversing memory loss. As the PKC activator macrocyclic lactone, e. g. a bryostatin, a benzolactam or a pyrollidone may be used.

### SUMMARY OF THE INVENTION

The present invention provides a pharmaceutical composition comprising a protein kinase C (PKC) activator as defined in claim 1 and a pharmaceutically acceptable carrier for use in a medicament effective to treat at least one symptom of stroke, wherein the administration according to claim 1 is applied.

The PKC activator is a macrocyclic lactone which is a bryostatin selected from bryostatin-1 , 2, 3, 4, 5, 6, 7, 8, 9, 10, 1 1, 12, 13, 14, 15, 16, 17 or 18. More preferably, the bryostatin is bryostatin-1.

Administration of the pharmaceutical compositions of the present invention is initiated at a time period chosen from within 1 day, within 2 days, within 3 days, between 1 and 2 days, and between 1 and 3 days of said stroke. In another embodiment, the administration of the pharmaceutical compositions of the present invention is initiated within 1, 2, 3, 4, 5, 6, 7, 8, 9, 10, 11 , 12, 13, 14, 15, 16, 17, 18, 19, 20, 21, 22, 23, or 24 hours of said stroke. In yet another embodiment, the administration of the pharmaceutical compositions of the present invention is initiated between 1 and 3, 1 and 5, 1 and 10, 1 and 24, 3 and 5, 3 and 10, 3 and 24, 5 and 10, 5 and 24, or 10 and 24 hours after said stroke. In yet another embodiment, the administration of the pharmaceutical compositions of the present invention is initiated after 3, 4, 5, 6, 7, 8, 9, 10, 11 , 12, 13, 14, 15, 16, 17, 18, 19, 20, 21, 22, 23, or 24 hours after said stroke/ischemic event. In yet another embodiment, the administration of the pharmaceutical compositions of the present invention is initiated after 1, 2, or 3 days after said stroke/ischemic event.

In one embodiment, treatment comprising the administration of the pharmaceutical compositions of the present invention is continued for a duration of about 1, 2, 3, 4, 5, 6, 7, 8, 9, 10, 11, or 12 weeks.

### BRIEF DESCRIPTION OF THE FIGURE

**Figure 1** depicts a spatial water maze performance of rats over training trials. Data are shown as means ± SEM. Bry, bryostatin-1 ; Isch, cerebral ischemia; MCDA, 4-methylcatechol-diacetic acid.
**Figure 2** depicts target quadrant ratio during probe test. Bry, bryostatin-1 ; Isch, ischemia; MCDA, 4-methylcatechol-diacetic acid *: p < 0.05. NS: p > 0.05.

### DETAILED DESCRIPTION OF THE INVENTION

### A. Definitions

As used herein, "administration" of a composition includes any route of administration, including oral subcutaneous, intraperitoneal, and intramuscular.

As used herein, "an effective amount" is an amount sufficient to reduce one or more symptoms associated with a stroke.

As used herein, "protein kinase C activator" or "PKC activator" means a substance that increases the rate of the reaction catalyzed by protein kinase C by binding to the protein kinase C.

As used herein, the term "subject" means a mammal.

As used herein, the term "pharmaceutically acceptable carrier" means a chemical composition with which the active ingredient may be combined and which, following the combination, can be used to administer the active ingredient to a subject. As used herein, the term "physiologically acceptable" ester or salt means an ester or salt form of the active ingredient which is compatible with any other ingredients of the pharmaceutical composition, which is not deleterious to the subject to which the composition is to be administered.

As used herein, "pharmaceutically acceptable carrier" also includes, but is not limited to, one or more of the following: excipients; surface active agents; dispersing agents; inert diluents; granulating and disintegrating agents; binding agents; lubricating agents; sweetening agents; flavoring agents; coloring agents; preservatives; physiologically degradable compositions such as gelatin; aqueous vehicles and solvents; oily vehicles and solvents; suspending agents; dispersing or wetting agents; emulsifying agents, demulcents; buffers; salts; thickening agents; fillers; emulsifying agents; antioxidants; antibiotics; antifungal agents; stabilizing agents; and pharmaceutically acceptable polymeric or hydrophobic materials. Other "additional ingredients" which may be included in the pharmaceutical compositions of the invention are known in the art and described, for example in Genaro, ed., 1985, Remington's Pharmaceutical Sciences, Mack Publishing Co., Easton, Pa., which is incorporated herein by reference.

The formulations of the pharmaceutical compositions described herein may be prepared by any method known or hereafter developed in the art of pharmacology. In general, such preparatory methods include the step of bringing the active ingredient into association with a carrier or one or more other accessory ingredients, and then, if necessary or desirable, shaping or packaging the product into a desired single- or multi-dose unit.

Although the descriptions of pharmaceutical compositions provided herein are principally directed to pharmaceutical compositions which are suitable for ethical administration to humans, it will be understood by the skilled artisan that such compositions are generally suitable for administration to animals of all sorts. Modification of pharmaceutical compositions suitable for administration to humans in order to render the compositions suitable for administration to various animals is well understood, and the ordinarily skilled veterinary pharmacologist can design and perform such modification with merely ordinary, if any, experimentation. Subjects to which administration of the pharmaceutical compositions of the invention is contemplated include, but are not limited to, humans and other primates, and other mammals.

Despite progress toward the development of new therapeutic agents and availability of several animal models, there is still a pressing need for improved animal models for screening

### B. Protein Kinase C (PKC)

The PKC gene family consists presently of 11 genes which are divided into four subgroups: 1) classical PKCα, βi, β₂ (β₁ and β₂ are alternatively spliced forms of the same gene) and γ, 2) novel PKCδ, ε, η, and θ, 3) atypical PKCζ, λ, η and *i* and 4) PKC µ. PKC µ resembles the novel PKC isoforms but differs by having a putative transmembrane domain (reviewed by Blohe et al. (1994) Cancer Metast. Rev. 13: 411 ; Ilug et al. (1993) Biochem J. 291 : 329; Kikkawa et al. (1989) Ann. Rev. Biochem. 58: 31). The α, β₁, β₂ and γ isoforms are C²⁺, phospholipid and diacylglycerol-dependent and represent the classical isoforms of PKC, whereas the other isoforms are activated by phospholipid and diacylglycerol but are not dependent on Ca²⁺. All isoforms encompass 5 variable (V1 -V5) regions, and the α, β and γ isoforms contain four (C1-C4) structural domains which are highly conserved. All isoforms except PKC α, β and γ lack the C2 domain, the λ η and isoforms also lack nine of two cysteine-rich zinc finger domains in C1 to which diacylglycerol binds. The C1 domain also contains the pseudosubstrate sequence which is highly conserved among all isoforms, and which serves an autoregulartory function by blocking the substrate-binding site to produce an inactive conformation of the enzyme (House et al. (1987) Science 238, 1726).

Because of these structural features, diverse PKC isoforms are thought to have highly specialized roles in signal transduction in response to physiological stimuli (Nishizuka (1989) Cancer 10: 1892), as well as in neoplastic transformation and differentiation (Glazer (1994) Protein Kinase C, J. F. Kuo, ed., Oxford U. Press at pages 171-198). For a discussion of known PKC modulators see PCT/US97/08141, U.S. Patent Nos. 5,652,232; 6,080,784; 5,891,906; 5,962,498; 5,955,501 ; 5,891,870 and 5,962,504.

There is increasing evidence that the individual PKC isozymes play significant roles in biological processes which provide the basis for pharmacological exploitation. One is the design of specific (preferably, isozyme specific) activators of PKC. This approach is complicated by the fact that the catalytic domain is not the domain primarily responsible for the isozyme specificity of PKC. These may provide a way to override the effect of other signal transduction pathways with opposite biological effects. Alternatively, by inducing down-regulation of PKC after acute activation, PKC activators may cause long term antagonism. Bryostatin is currently in clinical trials as an anti-cancer agent. The bryostatins are known to bind to the regulatory domain of PKC and to activate the enzyme. Bryostatins are examples of isozyme-selective activators of PKC. (see for example WO 97/43268;). For a discussion of known PKC modulators see WO 97/43268 A1, U.S. Patent Nos. 5,652,232; 6,043,270; 6,080,784; 5,891 ,906; 5,962,498; 5,955,501 ; 5,891,870 and 5,962,504.

Several classes of PKC activators have been identified. Phorbol esters, however, are not suitable compounds for eventual drug development because of their tumor promotion activity, (Ibarreta et al. (1999) Neuro Report 10(5&6): 1035-40). Of particular interest are macrocyclic lactones (i.e. bryostatin class and neristatin class) that act to stimulate PKC. Of the bryostatin class compounds., bryostatin- 1 has been shown to activate PKC and proven to be devoid of tumor promotion activity. Bryostatin- 1, as a PKC activator, is also particularly useful since the dose response curve of bryostatin-1 is biphasic. Additionally, bryostatin-1 demonstrates differential regulation of PKC isozymes, including PKCα, PKCδ and PKCε. Bryostatin-1 has undergone toxicity and safety studies in animals and humans and is actively investigated as an anti-cancer agent. Bryostatin-1 's use in the studies has determined that the main adverse reaction in humans is myalgia. One example of an effective dose is 40 µg/m² per week by intravenous injection.

Macrocyclic lactones, and particularly bryostatin-1 is described in U.S. Patent 4,560,774. Macrocyclic lactones and their derivatives are described elsewhere in U.S. Patent 6,187,568, U.S. Patent 6,043,270, U.S. Patent 5,393,897, U.S. Patent 5,072,004, U.S. Patent 5,196,447, U.S. Patent 4,833,257, and U.S. Patent 4,611,066. The above patents describe various compounds and various uses for macrocyclic lactones including their use as an anti-inflammatory or anti-tumor agent. (Szallasi et al. (1994) Journal of Biological Chemistry 269(3): 21 18-24; Zhang et al. (1996) Caner Research 56: 802-808; Hennings et al. (1987) Carcinogenesis 8(9): 1343-1346; Varterasian et al. (2000) Clinical Cancer Research 6: 825-828; Mutter et al. (2000) Bioorganic & Medicinal Chemistry 8: 1841-1860).

As will also be appreciated by one of ordinary skill in the art, macrocyclic lactone compounds and their derivatives, particularly the bryostatin class, are amenable to combinatorial synthetic techniques and thus libraries of the compounds can be generated to optimize pharmacological parameters, including, but not limited to efficacy and safety of the compositions. Additionally, these libraries can be assayed to determine those members that preferably modulate α-secretase and/or PKC.

Combinatorial libraries high throughput screening of natural products and fermentation broths has resulted in the discovery of several new drugs. At present, generation and screening of chemical diversity is being utilized extensively as a major technique for the discovery of lead compounds, and this is certainly a major fundamental advance in the area of drug discovery. Additionally, even after a "lead" compound has been identified, combinatorial techniques provide for a valuable tool for the optimization of desired biological activity. As will be appreciated, the subject reaction readily lend themselves to the creation of combinatorial libraries of compounds for the screening of pharmaceutical, or other biological or medically-related activity or material-related qualities. A combinatorial library for the purposes of the present invention is a mixture of chemically related compounds, which may be screened together for a desired property; said libraries may be in solution or covalently linked to a solid support. The preparation of many related compounds in a single reaction greatly reduces and simplifies the number of screening processes that need to be carried out. Screening for the appropriate biological property may be done by conventional methods. Thus, the present invention also provides methods for determining the ability of one or more inventive compounds to bind to effectively modulate α-secretase and/or PKC.

A variety of techniques are available in the art for generating combinatorial libraries described below, but it will be understood that the present invention is not intended to be limited by the foregoing examples and descriptions. (See, for example, Blondelle et al. (1995) Trends Anal. Chem. 14: 83; U.S. Patents 5,359,1 15; 5,362,899; U.S. 5,288,514: PCT publication WO 94/08051 ; Chen et al. (1994) JACCS 1 6:266 1 : Kerr et al. (1993) JACCS I 1 5:252; PCT publications WO92/10092, WO93/09668; WO91/07087; and WO93/20242). Accordingly, a variety of libraries on the order of about 16 to 1,000,000 or more diversomers can be synthesized and screened for a particular activity or property.

The following Table summarizes structural characteristics of bryostatin- 1 which has two pyran rings and one 6-membered cyclic acetal.

| **Name** | **PKC Affin (nM)** | **MW** | **Description** |
|---|---|---|---|
| Bryostatin 1 | 1.35 | 988 | 2 pyran + 1 cyclic acetal + macrocycle |

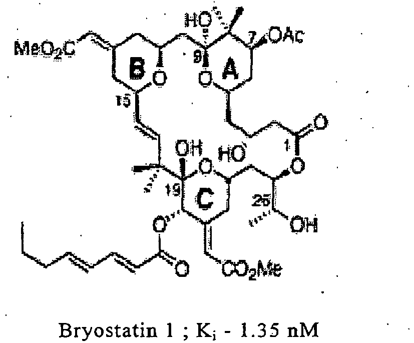

The compounds of the present invention can be administered by a variety of routes and in a variety of dosage forms including those for oral, rectal, parenteral (such as subcutaneous, intramuscular and intravenous), epidural, intrathecal, intra-articular, topical and buccal administration. The dose range for adult human beings will depend on a number of factors including the age, weight and condition of the patient and the administration route.

Reference to any compound herein includes the racemate as well as the single enantiomers.

### EXAMPLES

The following Examples serve to further illustrate the present invention.

### EXAMPLE 1 ; Global Ischemia Model of Stroke

Rats (male, Wistar, 200 - 225g) were randomly divided into 6 groups (8 each) and housed for 1 week before experimentation. Transient or permanent restriction of cerebral blood flow and oxygen supply results in ischemic stroke. The global ischemia model used to induce vascular memory impairment was two-vessel occlusion combined with a short term systemic hypoxia. Ligation of the bilateral common carotid arteries was performed under anesthesia (pentobarbital, 60 mg/kg, i.p.). After a one-week recovery from the surgery, rats were exposed to 14-min hypoxia (5% oxygen in a glass jar). Control rats (sham operated and vehicle controls) were subjected to the same incision to isolate both common carotid arteries and to 14-min air (in the glass jar). Body temperature was kept at 37-37.5 °C using a heating light source during the surgical procedure and until the animals were fully recovered.

### EXAMPLE 2: Bryostatin and MCDA Treatment

Bryostatin-1 was administered at 20 µg/m² (tail i.v., 2 doses/week, for 10 doses), starting 24 hours after the end of the hypoxic event. 4-Methylcatechol-diacetic acid (MCDA, a potential NGF and BDNF booster) was administered at 1.0 mg/kg (i.p., daily for the same 5-week period) in separate groups of rats.

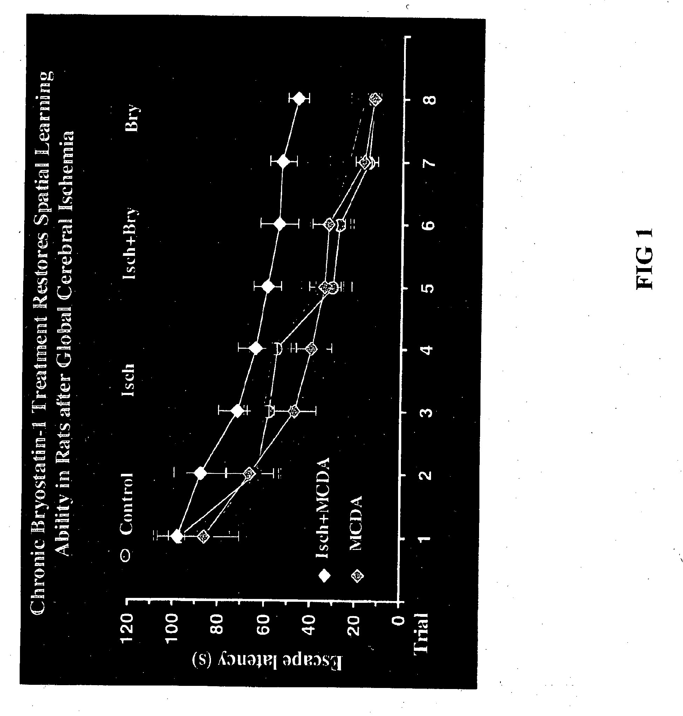

One week after the last bryostatin-1 , MCDA, or vehicle administration, rats were trained in the water maze spatial learning task (2 training trials per day for 4 days), followed by a probe test. A visible platform test was given after the probe test. The results are shown in **Figure 1****.**

Overall, there was a significant learning difference between the 6 groups (Figure 1 ; *F*_{5,383} = 27.480, p < 0.001 ; ANOVA). Detailed analysis revealed that the ischemic group did not learn the spatial maze task since there was no significant difference in escape latency over trials (*F*_{7,63} = 0.102, p > 0.05), a significantly impaired learning as compared with the control rats (group difference: *F*_{1,127} = 79.751, *p* < 0.001), while the rats in the other 5 groups all learned the task (the ischemic rats with MCDA treatment: *p* < 0.05 and the other 4 groups: *p* < 0.001 over trials). Bryostatin-1 therapy greatly improved the performance (Ischemic group with bryostatin-1 treatment vs. ischemic rats: *F*_{1,127} = 72.782, *p* < 0.001), to the level of performance that did not differ statistically from the control rats (Ischemic group with bryostatin-1 treatment vs. control rats: *F*_{1,127} = 0.001, *p* > 0.05). MCDA treatment also improved the learning of the ischemic rats (ischemia with NCDA treatment vs. ischemic rats: *F*_{1,127} = 15.584, *p* < 0.001) but the difference between the ischemia with MCDA treatment and control rats remained significant after the 5 week treatment (ischemia with NCDA treatment vs. control rats: *F*_{1,127} = 16.618, *p* < 0.001 ). There were no differences between the control and bryostatin-1 -only groups (bryostatin-1 vs. control: *F*_{1,127} = 0.010, *p >* 0.05) and between the control and MCDA-only groups (MCDA vs. control: *F*_{1,127} = 0.272, *p* > 0.05).

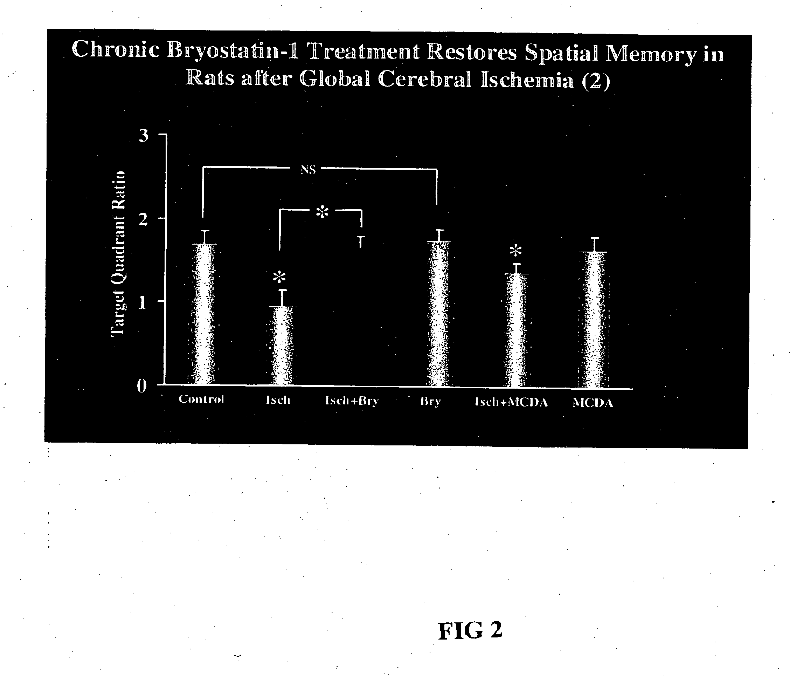

The rats in the ischemic group did not show a target preference in the probe test (F3,31 = 0.096, p > 0.05), while the rats of the other 5 groups all showed a target quadrant preference in the probe test (all p < 0.005). Data were analyzed using target quadrant ratio (dividing the target quadrant distance by the average of the non-target quadrant values during the probe test; Figure 2). There was a significant difference in the target quadrant ratios between the groups (F5,47 = 5.081, p < 0.001). Detailed analysis revealed group differences between the control and ischemic rats (F1, 15 = 9.451, p < 0.01), between the ischemic and ischemic with bryostatin-1 treatment (F1,15 = 10.328, p < 0.01), and between the ischemic with MCDA treatment and ischemic rats (F1, 15 = 5.623, p < 0.05), but no differences between the control and ischemic rats with bryostatin-1 treatment (F1 ,15 = 0.013, -p > 0.05), between the ischemic with MCDA treatment and control groups (F1,15 = 2.997, p > 0.05), between the control and bryostatin-1 -only rats (F1,15 = 0.064, p > 0.05), and between the control and the MCDA-only rats (F 1 , 15 = 0.0392, p >.0.05). A visible platform test, determined after the probe test revealed no significant difference between the groups (F5,47 = 0.115, p > 0.05), indicating that there were no significant group differences in sensorimotor ability of the rats.

### EXAMPLE 3: Bryostatin Treatment

Global cerebral ischemia/hypoxia was induced in male Wistar rats (225-250 g) by permanently occluding the bilateral common carotid arteries, combined with about 14 minutes of low oxygen (about 5%). Bryostatin- 1 was administered at 15 µg/m² (via a tail vein, 2 doses/week, for 10 doses), starting about 24 hours after the end of the ischemic/hypoxic event. Spatial learning (2 trials/ day for 4 days) and memory (a probe test of 1 minute, 24 hours after the last trial) task was performed 9 days after the last dose. Overall, there was a significant difference between the groups (F3,255 = 31.856, p<0.001) and groups x trials (F21,255 =1.648, p<0.05). Global cerebral ischemia impaired the spatial learning (ischemial vs. sham-operated F1,127 = 79.751 , p>0.001). The learning impairment was restored by Bryostatin-1 treatment (Bryostatin-1 + Ischemia vs. Ischemia: F1,127=50.233, p<0.001), while Bryostatin-1 alone did not affect the learning (Bryostatin-1 vs. sham-operated: F1,127 = 2.258, p>0.05; 9 days after the last dose).

In the memory retention test, sham-operated rats showed a target quadrant preference. Such good memory retention was not observed in the ischemic rats, indicating an impaired spatial memory. Bryostatin-1 therapy effectively restored memory retention after ischemia to the level of the sham-operated rats. Bryostatin-1 alone had no significant effects in the target quadrant preference compared with that of the sham-operated control rats. There was a significant difference in the quadrant ratios (calculated by dividing the target quadrant swim distance by the average swim distance in the non-target quadrants; F3,31 = 6.181, p<0.005) between the groups. Detailed analysis revealed significant differences between the ischemic rats and sham-operated control rats (F1,15 = 9.451 , p<0.01), between the ischemic rats and ischemic rats with Bryostatin-1 treatment (F1,15 10.328, p<0.01), but no significant differences between the ischemic rats with Bryostatin-1 treatment and sham-operated control (F 1,15 = 0.0131, p>0.05) and between the sham-operated control rats and Bryostatin-1 alone rats (F1,15 = 0.161, p>0.05). These results demonstrate that the cerebral ischemia/hypoxia produced an impairment of spatial learning and memory, tested about 7 weeks after the ischemic event. The impairment was lasting and not recoverable, during the time frame without appropriate intervention, but restored by chronic Bryostatin-1 treatment, even when the treatment was started 24 hours after the ischemic event, a wide therapeutic time-window.

## Claims

1. A pharmaceutical composition comprising a protein kinase C (PKC) activator and a pharmaceutically acceptable carrier for use in a medicament effective to treat at least one symptom of stroke, wherein an amount of said pharmaceutical composition effective for treatment of at least one symptom of stroke is administered to a patient, and the administration is initiated at a time period chosen from within 1 day, within 2 days, within 3 days, between 1 and 2 days, and between 1 and 3 days of the stroke, wherein said PKC activator is bryostatin-1, 2, 3, 4, 5, 6, 7, 8, 9, 10, 11, 12, 13, 14, 15, 16, 17, or 18.

2. The pharmaceutical composition of claim 1, wherein the bryostatin is bryostatin-1.

3. The pharmaceutical composition of claim 1, wherein the treatment is continued for a duration of time chosen from 1 week, 2 weeks, 3 weeks, 4 weeks, and 6 weeks.

4. The pharmaceutical composition of claim 1, wherein the treatment reverses stroke-induced brain injury.

5. The pharmaceutical composition of claim 1, wherein the treatment reverses stroke-induced memory impairment.

## Patentansprüche

1. Pharmazeutische Zusammensetzung, umfassend einen Proteinkinase C (PKC) -Aktivator und einen pharmazeutisch akzeptablen Träger, zur Verwendung in einem Medikament, das wirksam ist, um zumindest ein Symptom eines Schlaganfalls zu behandeln, wobei eine für die Behandlung von zumindest einem Symptom eines Schlaganfalls wirksame Menge der besagten pharmazeutischen Zusammensetzung an einen Patienten verabreicht wird und mit der Verabreichung innerhalb einer Zeitspanne von ausgewählt aus 1 Tag, 2 Tagen, 3 Tagen, zwischen 1 und 2 Tagen und zwischen 1 und 3 Tagen nach dem Schlaganfall begonnen wird, wobei der besagte PKC-Aktivator Bryostatin-1, 2, 3, 4, 5, 6, 7, 8, 9, 10, 11, 12, 13, 14, 15, 16, 17 oder 18 ist.

2. Pharmazeutische Zusammensetzung nach Anspruch 1, wobei das Bryostatin Bryostatin-1 ist.

3. Pharmazeutische Zusammensetzung nach Anspruch 1, wobei die Behandlung für eine Dauer, die aus 1 Woche, 2 Wochen, 3 Wochen, 4 Wochen und 6 Wochen ausgewählt ist, fortgesetzt wird.

4. Pharmazeutische Zusammensetzung nach Anspruch 1, wobei die Behandlung eine schlaganfallinduzierte Gehirnschädigung rückgängig macht.

5. Pharmazeutische Zusammensetzung nach Anspruch 1, wobei die Behandlung eine schlaganfallinduzierte Gedächtnisbeeinträchtigung rückgängig macht.

## Revendications

1. Composition pharmaceutique comprenant un activateur de la protéine kinase C (PKC) et un véhicule pharmaceutiquement acceptable destinée à être utilisée dans un médicament efficace pour traiter au moins un symptôme de l'AVC, une quantité de ladite composition pharmaceutique efficace pour le traitement d'au moins un symptôme de l'AVC étant administrée à un patient et l'administration débutant à une période choisie entre moins de 1 jour, moins de 2 jours, moins de 3 jours, entre 1 et 2 jours, et entre 1 et 3 jours après l'AVC, ledit activateur de la PKC étant la bryostatine 1, 2, 3, 4, 5, 6, 7, 8, 9, 10, 11, 12, 13, 14, 15, 16, 17 ou 18.

2. Composition pharmaceutique selon la revendication 1, où la bryostatine est la bryostatine 1.

3. Composition pharmaceutique selon la revendication 1, où le traitement est poursuivi pendant une durée choisie parmi 1 semaine, 2 semaines, 3 semaines, 4 semaines et 6 semaines.

4. Composition pharmaceutique selon la revendication 1, où le traitement inverse une lésion cérébrale induite par un AVC.

5. Composition pharmaceutique selon la revendication 1, où le traitement inverse une altération de la mémoire induite par un AVC.
